# EUROPÄISCHE PATENTSCHRIFT

(11) **EP 0 719 356 B1**
(45) Veröffentlichungstag und Bekanntmachung des Hinweises auf die Patenterteilung: **08.04.1998**
(21) Anmeldenummer: 94927538.2
(22) Anmeldetag: 02.09.1994
(51) Int. Cl.: D06L 3/12

(54) **AUFHELLERMISCHUNGEN AUF BASIS VON BISSTYRYLVERBINDUNGEN**
BRIGHTENING MIXTURES BASED ON BISTYRYL COMPOUNDS
MELANGES DE BLANCHIMENT OPTIQUE A BASE DE COMPOSES BISTYRYLE

(30) Priorität: 13.09.1993 DE 4330968
(43) Veröffentlichungstag der Anmeldung: 03.07.1996
(73) Patentinhaber: BASF AKTIENGESELLSCHAFT, 67056 Ludwigshafen (DE)
(72) Erfinder: HAUPTREIF, Manfred, D-76831 Birkweiler (DE); LEPPERT, Norbert, D-67346 Speyer (DE); EIZBACH, Karl-Heinz, D-67227 Frankenthal (DE); REICHELT, Helmut, D-67435 Neustadt (DE); RAATZ, Peter, D-67069 Ludwigshafen (DE); HERRMANN, Manfred, D-67061 Ludwigshafen (DE)
(86) Internationale Anmeldenummer: EP9402914
(87) Internationale Veröffentlichungsnummer: WO9508017

(56) Entgegenhaltungen:
- EP-A- 0 023 027
- EP-A- 0 030 917
- FR-A- 1 415 977
- GB-A- 2 200 660

## Beschreibung

Die vorliegende Erfindung betrifft neue Aufhellermischungen, enthaltend 75 Gew.-% bis 100 Gew.-%, bezogen auf das Gewicht der Mischung, der Bisstyrylverbindungen der Formeln I und II 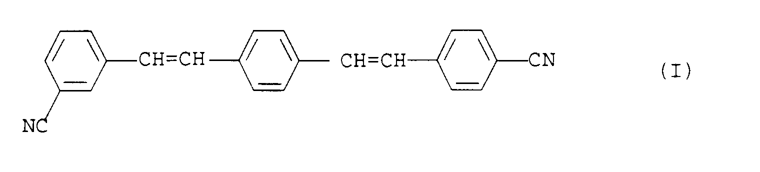 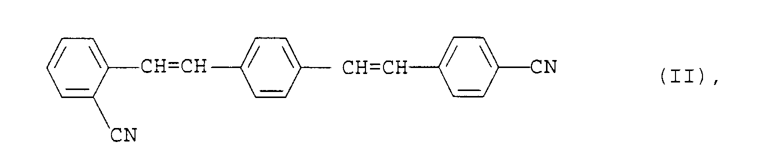 wobei die Bisstyrylverbindungen I und II im Gewichtsverhältnis 20:80 bis 60:40 vorliegen, mit der Maßgabe, daß das Gewichtsverhältnis 51:49 ausgenommen ist, Zubereitungen, enthaltend die neuen Mischungen, sowie ihre Verwendung zum optischen Aufhellen von synthetischen, halbsynthetischen oder natürlichen Polymermaterialien.

Aus der EP-A-238 446 und der EP-A-321 393 sind bereits Mischungen von optischen Aufhellern bekannt, die die obengenannten Bisstyrylverbindungen I und II im Gewichtsverhältnis 80,25:19,75 und 80:20 enthalten. Es hat sich jedoch gezeigt, daß diese Mischungen noch Nachteile in ihren anwendungstechnischen Eigenschaften aufweisen.

Die GB-A-2 200 660 beschreibt Aufhellermischungen, die 51 bis 98,5 Gew.-% der Bisstyrylverbindung I und 48,5 bis 1 Gew.-% der Bisstyrylverbindung II enthalten.

Aufgabe der vorliegenden Erfindung war es, neue Aufhellermischungen auf Basis von Bisstyrylverbindungen bereitzustellen, die einfach zugänglich sind und über vorteilhafte anwendungstechnische Eigenschaften, insbesondere über einen hohen Weißgrad verfügen.

Demgemäß wurden die eingangs näher bezeichneten Aufhellermischungen gefunden.

Die neuen Aufhellermischungen enthalten, jeweils bezogen auf das Gewicht der Mischung, 75 bis 100 Gew.-%, vorzugsweise 80 bis 100 Gew.-% und insbesondere 85 bis 100 Gew.-% der Bisstyrylverbindungen der Formeln I und II.

Von besonderem technischen Interesse sind Aufhellermischungen, enthaltend, bezogen auf das Gewicht der Mischung, 85 bis 90 Gew.-% der Bisstyrylverbindungen der Formeln I und II.

Als (auf 100 Gew.-% zu ergänzende) Restmengen können die erfindungsgemäßen Aufhellermischungen in der Regel noch Verbindungen der Formeln III und/oder IV 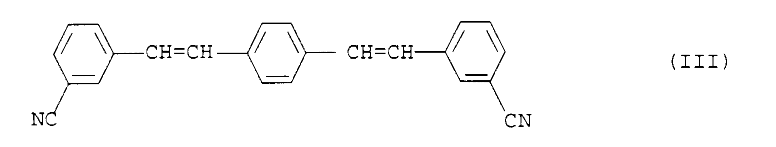 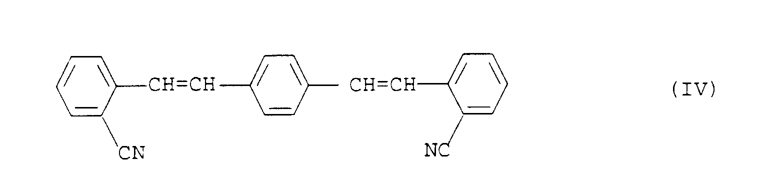 enthalten.

In den erfindungsgemäßen Aufhellermischungen liegen die Bisstyrylverbindungen der Formeln I und II im Gewichtsverhältnis 20:80 bis 60:40, vorzugsweise 30:70 bis 60:40 vor.

Von besonderem technischen Interesse sind Aufhellermischungen, in denen die Bisstyrylverbindungen der Formeln I und II im Gewichtsverhältnis 40:60 bis 50:50, insbesondere ca. 45:55, vorliegen.

Die neuen Aufhellermischungen können z.B. durch Mischen der Einzelkomponenten im entsprechenden Gewichtsverhältnis hergestellt werden. Die Einzelkomponenten können nach an sich bekannten Methoden erhalten werden. Beispielsweise kann man Terephthalaldehyd in einem inerten organischen Verdünnungsmittel in basischem Milieu mit Cyanophosphonsäurealkylestern umsetzen

Ein weiterer Gegenstand der vorliegenden Erfindung sind Aufhellerzubereitungen, enthaltend Wasser und, jeweils bezogen auf das Gewicht der Zubereitung, 3 bis 25 Gew.-%, vorzugsweise 5 bis 15 Gew.-%, der oben näher bezeichneten Aufhellermischung sowie 5 bis 60 Gew.-%, vorzugsweise 5 bis 52 Gew.-%, an Hilfsmitteln.

Geeignete Hilfsmittel sind z.B. anionische oder nichtionische Dispergiermittel, aus der Klasse der Ethylenoxidaddukte mit Fettalkoholen, höheren Fettsäuren oder Alkylphenolen oder Ethylendiamin-Ethylenoxid-Propylenoxidaddukte, oder Dispergiermittel, wie in der DE-A-2 745 449 beschrieben, Copolymerisate von N-Vinylpyrrolidon mit 3-Vinylpropionsäure, Wasserrückhaltemittel, wie Ethylenglykol, Glycerin oder Sorbit, oder Biocide.

Eine bevorzugte Aufhellerzubereitung enthält neben Wasser, jeweils bezogen auf das Gewicht der Zubereitung, 3 bis 25 Gew.-%, vorzugsweise 5 bis 15 Gew.-%, der oben näher bezeichneten Aufhellermischung, 3 bis 12 Gew.-% anionisches oder nichtionisches Dispergiermittel, 1 bis 15 Gew.-% Copolymerisate von N-Vinylpyrrolidon mit 3-Vinylpropionsäure und 1 bis 25 Gew.-% weiterer Hilfsmittel (z.B. Wasserrückhaltemittel oder Biocide).

Die erfindungsgemäßen Aufhellermischungen eignen sich in vorteilhafter Weise zum optischen Aufhellen von synthetischen, halbsynthetischen oder natürlichen Polymermaterialien, vorzugsweise von Polyester und insbesondere von Polyestergeweben. Sie weisen bei niedriger Fixier- oder Ausziehtemperatur günstige Applikationseigenschaften auf. Außerdem besitzen sie eine hohe Ausgiebigkeit.

Die folgenden Beispiele sollen die Erfindung näher erläutern.

### Herstellung der Aufhellermischung

a) In 1100 ml N,N-Dimethylformamid wurden 201 g (1,47 mol) Terephthalaldehyd (98,45 Gew.-%ig) und 402,7 g (1,47 mol) 3-Cyanobenzylphosphonsäurediethylester (91,7 gew.-%ig) vorgelegt. Zu dieser Mischung wurden innerhalb von 4 h bei 30°C 279,8 g (1,54 mol) 30 gew.-%ige methanolische Natriummethanolatlösung zugegeben. Danach wurde 2 h bei 30°C nachgerührt, und anschließend wurden 434,9 g (1,54 mol) 4-Cyanobenzylphosphonsäurediethylester und schließlich weitere 279,8 g (1,54 mol) 30 gew.-%ige methanolische Natriummethanolatlösung zugegeben. Nachdem 4 h bei 30°C nachgerührt wurde, kühlte man auf 25°C ab, saugte den angefallenen Niederschlag ab, wusch ihn dreimal mit je 500 ml Methanol und einmal mit 1650 ml Wasser und trocknete ihn.
   Man erhielt 335 g einer Mischung, die 284 g der Verbindung der Formel 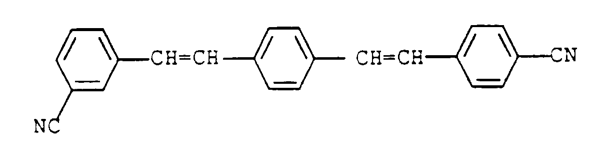 und 34 g der Verbindung der Formel 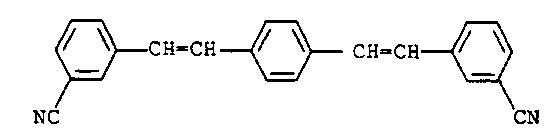 enthielt.
b) In 1100 ml N,N-Dimethylformamid wurden 201 g (1,47 mol) Terephthalaldehyd (98,45 Gew.-%ig) und 395,7 g (1,47 mol) 2-Cyanobenzylphosphonsäurediethylester (94 gew.-%ig) vorgelegt. Zu dieser Mischung wurden innerhalb von 3 h bei 30°C 279,8 g (1,54 mol) 30 gew.-%ige methanolische Natriummethanolatlösung zugegeben. Danach wurde 1 h bei 30°C nachgerührt, und anschließend wurden 434,9 g (1,54 mol) 4-Cyanobenzylphosphonsäurediethylester und schließlich weitere 279,8 g (1,54 mol) 30 gew.-%ige methanolische Natriummethanolatlösung zugegeben. Nachdem 1 h bei 30°C nachgerührt wurde, kühlte man auf 25°C ab, saugte den angefallenen Niederschlag ab, wusch ihn dreimal mit je 500 ml Methanol und einmal mit 1650 ml Wasser und Trocknete ihn.
   Man erhielt 332 g einer Mischung, die 279 g der Verbindung der Formel 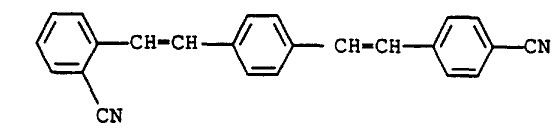 und 49 g der Verbindung der Formel 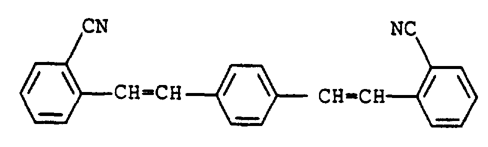 enthielt.
c) Die unter a) und b) beschriebenen Komponenten wurden jeweils im Gewichtserhältnis 30:70 (Beispiel 1), 45:45 (Beispiel 2) und 68:32 (Beispiel 3) gemischt.

### Herstellung der Zubereitung (allgemeine Vorschrift)

11 Gew.-Teile der unter c) beschriebenen Aufhellermischung, 35 Gew.-% des Dispergiermittels, das in der DE-A-2 745 449 in Beispiel 13 beschrieben ist, 5 Gew.-Teile Copolymerisat von N-Vinylpyrrolidon mit 3-Vinylpropionsäure, 12 Gew.-Teile Glycerin, 0,5 Gew.-Teile 30 gew.-%ige wäßrige Formaldehydlösung und 68 Gew.-Teile Wasser werden bei einem pH-Wert von 8 solange in einer Rührwerkkugelmühle vermahlen, bis die Teilchengröße des Aufhellers kleiner als 5 µm ist. Man erhält dabei dünnflüssige Dispersionen, die lagerstabil sind und keine Sedimentation aufweisen.

### Allgemeine Färbevorschrift

a) HT-Verfahren
   Polyestergewebe wird bei 55°C in ein Färbebad eingebracht, das x Gew.-% Aufheller-Zubereitung (bezogen auf das Gewicht des Gewebes) und 1 g/l des Natriumsalzes eines Kondensationsproduktes aus Naphthalin-2-sulfonsäure und Formaldehyd enthält und dessen pH-Wert durch Zusatz von Essigsäure auf 5 bis 5,5 gestellt wurde. Das Bad wird dann innerhalb von 30 Minuten auf 130°C erhitzt und noch 30 Minuten bei dieser Temperatur gehalten. Danach wird das Gewebe gespült und getrocknet.
   Die Konzentration an Aufhellerzubereitung (enthaltend die jeweilige Aufhellermischung aus den Beispielen 1 bis 3) betrug jeweils 0,22 Gew.-% und 1,0 Gew.-%. In allen Fällen wurden gute Aufhelleffekte erzielt.
b) Thermosolverfahren
   Man foulardiert bei Raumtemperatur Polyestergewebe mit einer wäßrigen Flotte, enthaltend x g/l Aufhellerzubereitung. Die Flottenaufnahme beträgt 60 %. Anschließend wird das Gewebe getrocknet und danach bei 190°C für 30 Sekunden fixiert.
   Die Konzentration an Aufhellerzubereitung (enthaltend die jeweiligen Aufhellermischungen aus den Beispielen 1 bis 3) betrug jeweils 2,7 g/l und 10 g/l. In allen Fällen wurden gute Aufhelleffekte erzielt.

## Patentansprüche

1. Aufhellermischungen, enthaltend 75 Gew.-% bis 100 Gew.-%, bezogen auf das Gewicht der Mischung, der Bisstyrylverbindungen der Formeln I und II 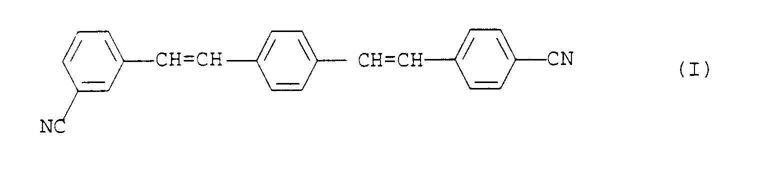 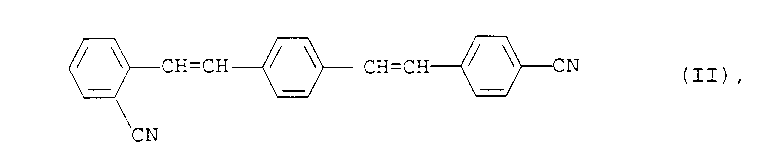 wobei die Bisstyrylverbindungen I und II im Gewichtsverhältnis 20:80 bis 60:40 vorliegen, mit der Maßgabe, daß das Gewichtsverhältnis 51:49 ausgenommen ist.

2. Aufhellermischungen nach Anspruch 1, enthaltend 80 bis 100 Gew.-%, bezogen auf das Gewicht der Mischung, der Bisstyrylverbindungen der Formeln I und II.

3. Aufhellermischungen nach Anspruch 1, enthaltend 85 bis 100 Gew.-%, bezogen auf das Gewicht der Mischung, der Bisstyrylverbindungen der Formeln I und II.

4. Aufhellermischungen nach Anspruch 1, enthaltend die Bisstyrylverbindungen der Formeln I und II im Gewichtsverhältnis 30:70 bis 60:40.

5. Aufhellerzubereitungen, enthaltend Wasser und, jeweils bezogen auf das Gewicht der Zubereizung, 3 bis 25 Gew.-% der Aufhellermischung gemäß Anspruch 1 sowie 5 bis 60 Gew.-% an Hilfsmitteln.

6. Verwendung der Aufhellermischungen gemäß Anspruch 1 zum optischen Aufhellen von synthetischen, halbsynthetischen oder natürlichen Polymermaterialien.

## Claims

1. Brightener mixtures containing from 75% by weight to 100% by weight, based on the weight of the mixture, of the bisstyryl compounds of the formulae I and II 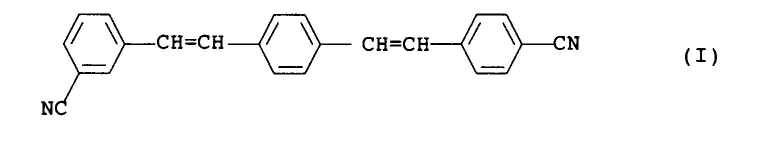 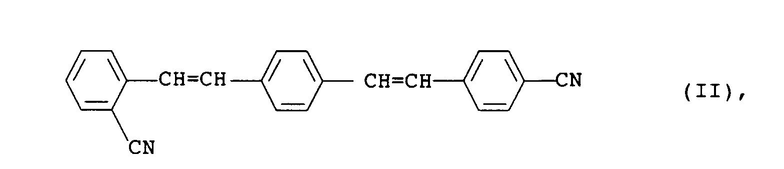 in a weight ratio from 20:80 to 60:40, with the proviso that the weight ratio of 51:49 is excluded.

2. Brightener mixtures as claimed in claim 1 containing from 80 to 100% by weight, based on the weight of the mixture, of the bisstyryl compounds of the formulae I and II.

3. Brightener mixtures as claimed in claim 1 containing from 85 to 100% by weight, based on the weight of the mixture, of the bisstyryl compounds of the formulae I and II.

4. Brightener mixtures as claimed in claim 1 containing the bisstyryl compounds of the formulae I and II in a weight ratio from 30:70 to 60:40.

5. Brightener formulations containing water and, in each case based on the weight of the formulation, from 3 to 25% by weight of the brightener mixture of claim 1 and from 5 to 60% by weight of auxiliaries.

6. The use of the brightener mixtures of claim 1 for optically brightening synthetic, semisynthetic or natural polymer materials.

## Revendications

1. Mélanges de blanchiment optique, contenant 75 à 100% en poids, par rapport au poids du mélange, des dérivés de bisstyryle de formules I et II 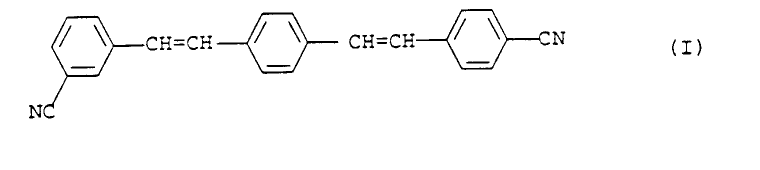 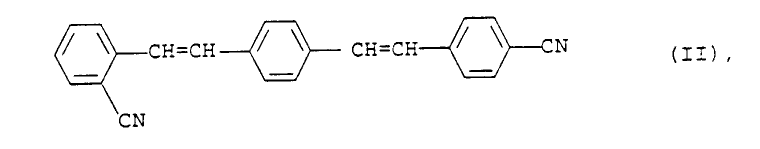 les dérivés de bisstyryle I et II étant présents dans un rapport molaire de 20 : 80 à 60 : 40, pourvu que le rapport molaire 51 : 49 soit exclu.

2. Mélanges de blanchiment optique selon la revendication 1, contenant 80 à 100%, par rapport au poids du mélange, des dérivés de bisstyryle de formules I et II.

3. Mélanges de blanchiment optique selon la revendication 1, contenant 85 à 100% en poids, par rapport au poids du mélange, des dérivés de bisstyryle de formules I et II.

4. Mélanges de blanchiment optique selon la revendication 1, contenant les dérivés de bisstyryle de formule I et II dans un rapport molaire de 30 : 70 à 60 : 40.

5. Préparations de blanchiment optique, contenant de l'eau et, à chaque fois par rapport au poids de la préparation, 3 à 25% en poids du mélange de blanchiment optique selon la revendication 1 ainsi que 5 à 60% en poids d'adjuvants.

6. Utilisation des mélanges de blanchiment optique selon la revendication 1, pour le blanchiment optique de matériaux polymères synthétiques, semi-synthétiques ou naturels.
